# EUROPEAN PATENT APPLICATION

(11) **EP 4 767 934 A1**
(43) Date of publication of application: **01.07.2026**
(21) Application number: 24860351.6
(22) Date of filing: 26.08.2024
(51) Int. Cl.: A61B 5/145

(54) **ANALYTE MONITORING DEVICE**

(30) Priority: 25.08.2023 KR 20230111913
(71) Applicant: SD Biosensor, Inc., Gyeonggi-do 16690 (KR)
(72) Inventor: LEE, Hyo Keun, Suwon-si, Gyeonggi-do 16690 (KR); PARK, Hyo Lim, Suwon-si, Gyeonggi-do 16684 (KR); HONG, Soon Min, Hwaseong-si, Gyeonggi-do 18477 (KR); JEON, Sang Yeun, Yongin-si, Gyeonggi-do 17091 (KR); LEE, Sang Min, Suwon-si, Gyeonggi-do 16690 (KR)
(74) Representative: Eisenführ Speiser
(86) International application number: PCT/KR2024/012703
(87) International publication number: WO 2025/048431

(57) **Abstract**

An analyte monitoring device according to one embodiment of the present invention comprises: a housing; a substrate provided inside the housing; a needle unit including a needle body provided to be movable inside the housing, and a needle provided at the lower end of the needle body and inserted into a physical body so as to penetrate a sensor; and a sensing unit which is arranged on the inside and/or the outside of the housing, and which is electrically conducting while in contact with the needle unit, wherein, when the needle unit is triggered, and then separated from the sensing unit, a wake-up trigger for power source supply can be transmitted to the substrate.

## Description

### [Technical Field]

The present invention relates to an analyte monitoring device, and more particularly, to a device inserted into the body to monitor an analyte.

### [Background Art]

As preferences for processed foods have increased in recent years, people are easily exposed to monosaccharides. Under these circumstances, diabetes is also rapidly increasing. When blood glucose levels rapidly drop or rise in diabetic patients, it may lead to shock and, in rare cases, death. Accordingly, diabetic patients need to continuously monitor their blood glucose levels.

In the case of conventional blood glucose measuring devices, a fingertip is cut using a lancet, and the blood of a diabetic patient that comes out through the incision is inserted into a blood glucose analyzer to calculate a blood glucose level.

This type of blood glucose measuring device causes pain and fear in a user. In addition, it is unrealistic to prick one's finger every hour to cause bleeding.

In order to solve these problems, continuous glucose monitoring (CGM) devices have been developed. When a sensor having a very small thickness and an electronic device for analyzing an analyte (hereinafter "blood glucose") measured by the sensor are attached to the skin of a diabetic patient, blood glucose is continuously measured for a period of one to two weeks.

By being inserted under the skin, the continuous glucose monitoring device may continuously measure blood glucose levels with a sensor provided therein. In general, there are ongoing efforts to miniaturize the continuous glucose monitoring device so as to be inserted under the skin.

In the case of the conventional continuous glucose monitoring device, a semiconductor sensor or a mechanical switch has been employed for supplying power to a battery. However, when the semiconductor sensor is applied, additional battery consumption is required, and when the mechanical switch is used instead of the semiconductor sensor, there is a problem that mechanical failure occurs when the mechanical switch is stored in a pressed state for a long period of time.

### [Description of Invention]

### [Technical Problem]

One embodiment of the present invention is directed to providing an analyte monitoring device capable of automatically supplying power from a point in time of product use and improving device durability during storage and use, by utilizing the contact and separation of a sensing unit and a conductive portion made of a conductive material, without applying a semiconductor sensor or a mechanical switch, when supplying power to the analyte monitoring device.

Problems to be solved by the present invention are not limited to those mentioned above, and other problems that have not been mentioned will be clearly understood by those skilled in the art from the following description.

### [Technical Solution]

An analyte monitoring device according to one embodiment of the present invention includes a housing, a substrate installed inside the housing, a needle unit including a needle body movably installed inside the housing and a needle provided at a lower end of the needle body and inserted into a body to invasively introduce a sensor therein, and a sensing unit disposed on at least one of an inner side or an outer side of the housing and formed to become electrically conductive in a contact state with the needle unit, wherein, when the needle unit is separated from the sensing unit after triggered, a wake-up trigger for power supply is transmitted to the substrate.

The sensing unit may be made of a quantum tunneling composite (QTC).

The sensing unit may be made in a cylindrical shape or flat shape with a predetermined volume.

A holder to which the needle unit is fixed may be provided inside the housing, and the sensing unit may be provided to surround the holder.

An analyte monitoring device according to another embodiment of the present invention includes a housing, a substrate installed inside the housing, a needle unit including a needle body movably installed inside the housing and a needle provided at a lower end of the needle body and inserted into a body to invasively introduce a sensor therein, a first conductive portion disposed on a lower surface of the needle body, and a second conductive portion disposed on at least one of an inner side or an outer side of the housing and formed to become electrically conductive in a contact state with the needle unit, wherein, when the needle unit is separated from the second conductive portion after triggered, a wake-up trigger for power supply is transmitted to the substrate.

The first conductive portion and the second conductive portion may be made of conductive silicone rubber.

An analyte monitoring device according to still another aspect of the present invention includes a housing, a needle unit including a needle body movably installed inside the housing and a needle provided at a lower end of the needle body and inserted into a body to invasively introduce a sensor therein, a first terminal portion provided on the housing, a transmitter holder to which the needle unit is fixedly installed, which is coupled to an upper portion of the housing, and which includes a second terminal portion that comes into contact with the first terminal portion when coupled to the upper portion of the housing, and a substrate installed inside the housing, electrically connected to the first terminal portion, and configured to transmit a wake-up trigger for power supply when the first terminal portion and the second terminal portion are separated after the needle unit is triggered.

A cutout in which a needle coupling portion to which the needle unit is coupled is disposed may be formed in the housing.

A holder for fixing the needle unit may be provided in the needle coupling portion.

The housing may include a lower housing and an upper housing coupled to an upper portion of the lower housing, and the first terminal portion may be disposed across an upper surface, an inner side surface, and a lower surface of the upper housing.

The first terminal portion may include a 1-1 terminal disposed on the upper surface of the upper housing, a 1-2 terminal extending from the 1-1 terminal and disposed on a side surface of the cutout, and a 1-3 terminal extending from the 1-2 terminal and disposed on the lower surface of the upper housing.

The 1-3 terminal may be electrically connected to the second terminal portion.

The first terminal portion may be patterned by a laser direct structuring (LDS) method.

An analyte monitoring device according to yet another embodiment of the present invention includes a housing, a needle unit including a needle body movably installed inside the housing and a needle provided at a lower end of the needle body and inserted into a body to invasively introduce a sensor therein, a first terminal portion provided on the housing, a second terminal portion provided on a lower surface of the needle unit and electrically connected to the first terminal portion, and a substrate installed inside the housing, electrically connected to the first terminal portion, and configured to transmit a wake-up trigger for power supply when the first terminal portion and the second terminal portion are separated after the needle unit is triggered.

A cutout in which a needle coupling portion to which the needle unit is coupled is disposed may be formed in the housing.

The housing may include a lower housing and an upper housing coupled to an upper portion of the lower housing, and the first terminal portion may be disposed across an upper surface, an inner side surface, and a lower surface of the upper housing.

The first terminal portion may include a 1-1 terminal disposed on the upper surface of the upper housing, a 1-2 terminal extending from the 1-1 terminal and disposed on a side surface of the cutout, and a 1-3 terminal extending from the 1-2 terminal and disposed on the lower surface of the upper housing.

The 1-3 terminal may be electrically connected to the second terminal portion.

The first terminal portion may be patterned by an LDS method.

### [Advantageous Effects]

According to one embodiment of the present invention, when supplying power to an analyte monitoring device, by utilizing the contact and separation of a sensing unit and a conductive portion, which are made of a conductive material, without applying a semiconductor sensor or a mechanical switch, power can be automatically suppled from a point in time of product use and device durability can be improved during storage and use.

### [Description of Drawings]

FIG. 1 is a view illustrating an analyte monitoring device according to one embodiment of the present invention.
FIG. 2 is a view illustrating a coupled state of a needle unit and a transmitter of the analyte monitoring device according to one embodiment of the present invention.
FIG. 3 is a view illustrating a separated state of the needle unit and the transmitter of the analyte monitoring device according to one embodiment of the present invention.
FIG. 4 is a view illustrating an analyte monitoring device according to another embodiment of the present invention.
FIG. 5 is a view illustrating a coupled state of a needle unit and a transmitter of the analyte monitoring device according to another embodiment of the present invention.
FIG. 6 is a view illustrating a separated state of the needle unit and the transmitter of the analyte monitoring device according to another embodiment of the present invention.
FIG. 7 is an exploded perspective view illustrating an analyte monitoring device according to still another embodiment of the present invention.
FIG. 8 is a view illustrating the analyte monitoring device according to still another embodiment of the present invention.
FIG. 9 is a view illustrating an upper surface of an upper housing of the analyte monitoring device according to still another embodiment of the present invention.
FIG. 10 is a view illustrating a lower surface of the upper housing of the analyte monitoring device according to still another embodiment of the present invention.
FIG. 11 is a view illustrating an analyte monitoring device according to yet another embodiment of the present invention.
FIG. 12 is a view illustrating a lower surface of an upper housing of the analyte monitoring device according to yet another embodiment of the present invention.
FIG. 13 is an exploded perspective view illustrating an analyte monitoring device according to yet another embodiment of the present invention.
FIG. 14 is a view illustrating a lower surface of a transmitter holder of the analyte monitoring device according to yet another embodiment of the present invention.
FIG. 15 is an exploded perspective view illustrating an analyte monitoring device according to yet another embodiment of the present invention.

### [Modes of the Invention]

Since the present invention may be variously modified and have several embodiments, specific embodiments thereof will be illustrated in the accompanying drawings and described in detail. However, this is not intended to limit the present invention to specific embodiments, and the present invention should be understood to include all modifications, equivalents, and substitutes included in the spirit and scope thereof. In describing the present invention, when it is determined that a detailed description of the related art may obscure the subject matter of the present invention, the detailed description thereof will be omitted.

While the terms "first," "second," etc., may be used herein to describe various elements, such elements should not be limited by the terms. These terms are only used to distinguish one element from another element.

Terms used in the present application are merely provided to describe specific embodiments and are not intended to limit the present invention. The singular forms are intended to include the plural forms as well, unless the context clearly indicates otherwise. In the present application, it will be understood that the terms "include," "have," and the like are intended to specify the presence of features, integers, steps, operations, elements, components, and/or combinations thereof stated in the specification, but do not preclude the possibility of the presence or addition of one or more other features, integers, steps, operations, elements, components, or combinations thereof.

In addition, throughout the specification, when "connected" is used, this does not necessarily mean two or more components are directly connected, but means that two or more components are indirectly connected through another component, that the components are not only physically connected, but also electrically connected, or that the components are integrated although the components are referred to by different names depending on a location or a function.

Further, when a component is described as being formed or disposed "on (above) or under (below)" another component, the term "on (above) or under (below)" includes not only when two components are in direct contact with each other, but also when one or more other components are formed or disposed between the two components. Further, when a component is described as being "on (above) or below (under)," the description may include the meanings of an upward direction and a downward direction based on one component.

Hereinafter, embodiments of an analyte monitoring device according to the present invention will be described in detail with reference to the accompanying drawings, and in the following description with reference to the accompanying drawings, identical or corresponding components will be assigned the same numbers and overlapping descriptions thereof will be omitted.

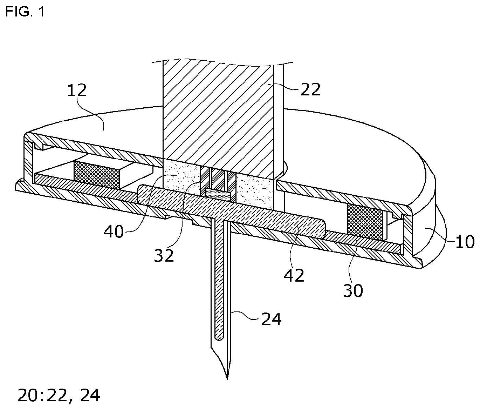

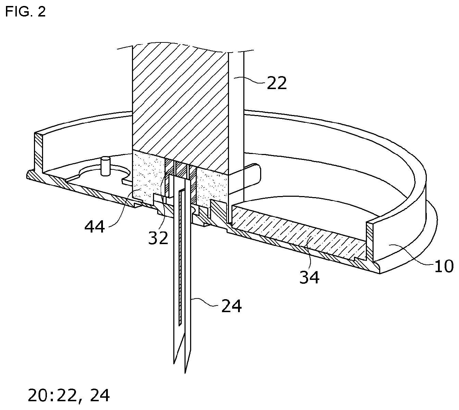

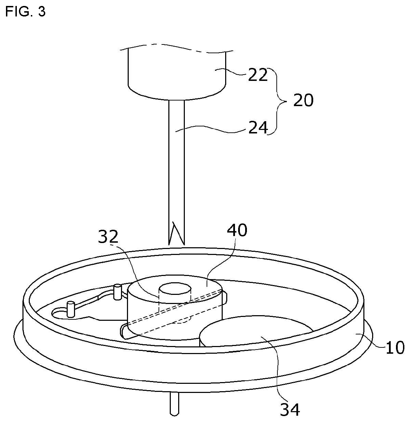

FIG. 1 is a view illustrating an analyte monitoring device according to one embodiment of the present invention, FIG. 2 is a view illustrating a coupled state of a needle unit and a transmitter of the analyte monitoring device according to one embodiment of the present invention, and FIG. 3 is a view illustrating a separated state of the needle unit and the transmitter of the analyte monitoring device according to one embodiment of the present invention.

As illustrated in FIGS. 1 to 3, an analyte monitoring device according to one embodiment of the present invention may include a housing 10, 12, a substrate 30 installed inside the housing 10, 12, a needle unit 20 including a needle body 22 movably installed inside the housing 10, 12 and a needle 24 provided at a lower end of the needle body 22 and inserted into a body, and a sensing unit 40 disposed on at least one of an inner side or an outer side of the housing 10, 12 and formed to become electrically conductive in a contact state with the needle unit 20, and when the needle unit 20 is separated from the sensing unit 40 after triggered, a wake-up trigger for power supply may be transmitted to the substrate 30.

The housing 10, 12 may include a lower housing 10 and an upper housing 12 coupled to the upper portion of the lower housing 10, but is not limited thereto, and the housings 10 and 12 may be integrally formed. An opening through which the needle 24 passes may be formed at a central portion of the lower housing 10. The lower housing 10, which is a surface that comes into contact with the body, may be made of a material that is deformed to fit the body, and an adhesive surface on which an adhesive is applied may be formed on an outer side surface of the lower housing 10 so that the analyte monitoring device may be attached to a body surface.

The upper housing 12 is coupled to the lower housing 10 to protect the various parts mounted in the lower housing 10. The upper housing 12 may be pressed by an applicator, that is, a tool on which the analyte monitoring device is mounted, to insert a sensor 42 of the analyte monitoring device into the user's body, and this pressing may allow the analyte monitoring device to be attached to the body. The upper housing 12 may be made of synthetic resin, for example, plastic, but is not limited thereto and may be made of various materials.

The needle unit 20 includes the needle body 22 and the needle 24 provided at a tip of the needle body 22. The needle unit 20 is linearly moved forward or backward by the applicator. When the needle unit 20 is triggered, the needle 24 may be inserted into the body and the sensor 42 disposed inside the needle 24 may be invasively introduced. The needle 24 incises the skin so that at least a portion of the tip may be invasively introduced into the body in an insertion direction, and guides the sensor 42 into the body. The sensor 42 is inserted into the body and measures information on an analyte in the body. The sensor 42 may be inserted into the body to measure information on analytes in the body, such as glucose, and transmit the measured information to the substrate 30 through the needle body 22.

The substrate 30 may be mounted on an inner surface of the lower housing 10 and achieves electrical connection between parts mounted on the substrate 30 and the sensor 42. The analyte information measured by the sensor 42 may be transmitted to the substrate 30, and the substrate 30 may process a transmitted electrical signal using a predetermined method. The substrate 30 is illustrated as being mounted in a circular shape on the inner surface of the lower housing 10 but is not limited thereto and may be formed in various shapes.

A holder 32 is coupled to the needle unit 20 and serves to fix the needle unit 20 to the lower housing 10. The holder 32 aligns the needle unit 20 fixed to the lower housing 10 to allow the needle 24 to be inserted vertically downward. In one embodiment, the holder 32 may be configured with a rubber packing to fix the needle unit 20. In one embodiment, the holder 32 may be formed so that the rubber packing surrounds only an edge portion.

A battery 34 may be connected to the substrate 30 and may supply power to the analyte monitoring device. In the drawing, the battery 34 is illustrated as being formed in a circular shape, but is not limited thereto and the battery 34 may have various shapes. In the present embodiment, the battery 34 may supply power to the substrate 30 by a wake-up trigger operation of the substrate 30 when the needle unit 20 is separated from the substrate 30 by triggering.

The sensing unit 40 may be mounted on the inner surface of the lower housing 10. The sensing unit 40 may be mounted on the inner surface of the lower housing 10 and may be disposed at a position corresponding to a position of the needle unit 20. That is, the sensing unit 40 may be disposed directly below the needle unit 20, and when the needle unit 20 advances, the sensing unit 40 may come into contact therewith and conduct electricity as illustrated in FIG. 3. In the case of the analyte monitoring device, before use, the needle unit 20 maintains a contact state with the sensing unit 40, and when used, the needle unit 20 is triggered by the applicator and ultimately separated from the sensing unit 40.

In one embodiment, the sensing unit 40 may be made of a quantum tunneling composite (QTC). The QTC is a flexible polymer exhibiting excellent electrical properties, making it ideal for use in a pressure sensor and a switching system, and is a composite material of metal and a non-conductive elastomeric binder for use in a pressure sensor. The QTC basically uses the principle of quantum tunneling, which is widely applied in applied physics, and a quantum tunneling composite sheet may include three layers: a QTC layer, a conductive layer, and an insulator. When a force is applied to a surface of the plastic insulator, electrons may tunnel from the conductive layer to the QTC layer, such that the QTC conducts electricity.

In the present embodiment, the QTC material is presented as one example of the sensing unit 40, but the sensing unit 40 is not limited thereto, and the sensing unit 40 may be made of any touch sensor material that is capable of transmitting a wake-up trigger signal by becoming electrically conductive upon coming into contact with the needle unit 20. In addition, in the present embodiment, the sensing unit 40 is illustrated as being mounted inside the lower housing 10, but is not limited thereto, and may be mounted outside the housing 10 or may be mounted inside and outside the housing 10.

In this way, when the sensing unit 40 comes into contact with the needle unit 20 as the needle unit 20 advances, the sensing unit 40 becomes electrically conductive, and in this case, the sensing unit 40 is in a switch-off state and is electrically disconnected from the substrate 30. Thereafter, when the analyte monitoring device is used, the needle unit 20 is triggered using the applicator, and the needle 24 is inserted into the body and guides the invasive introduction of the sensor 42. Then, the needle unit 20 is pulled out of the body and physically separated from the sensing unit 40. At this time, when pressure transmission from the needle unit 20 to the sensing unit 40 is released, the sensing unit 40 enters a switch-on state and transmits the wake-up trigger signal to the substrate 30. Then, power is supplied from the battery 34 to the substrate 30. In the miniaturized analyte monitoring device, it is necessary to automatically supply power, and since the battery 34 is activated through the triggering and separation operation of the needle unit 20, automatic power supply to the substrate 30 is enabled.

Meanwhile, the sensing unit 40 presented in the present embodiment may have a circular shape with a predetermined volume as illustrated in the drawing, but is not limited thereto. That is, any shape may be applied to the sensing unit 40 provided that the sensing unit 40 may come into contact with the needle unit 20 as the needle unit 20 advances. For example, the sensing unit 40 may have a cross-section such as a square, a triangle, or the like, or may have a flat shape without volume. In addition, a plurality of sensing units 40 may be disposed on the inner surface of the lower housing 10 and may come into contact with the needle unit 20. In addition, the sensing unit 40 may have a shape that surrounds the holder 32.

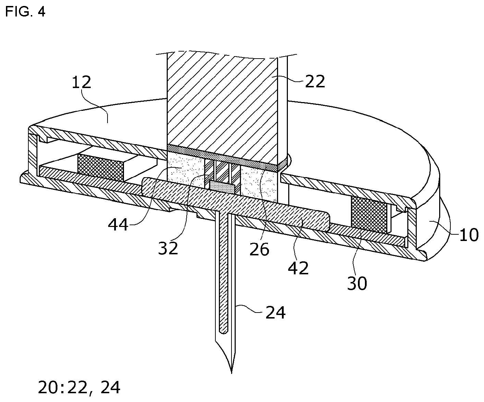

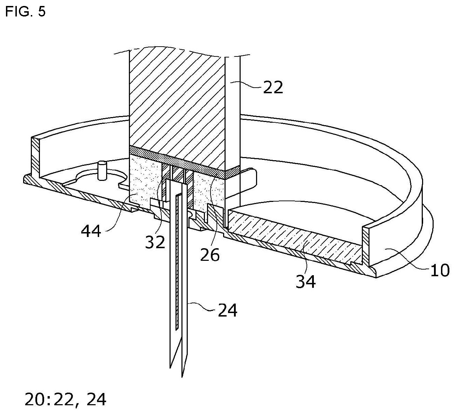

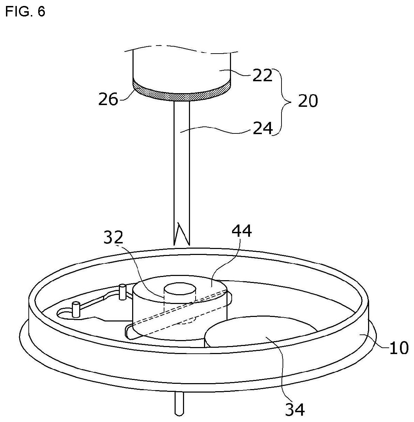

FIG. 4 is a view illustrating an analyte monitoring device according to another embodiment of the present invention, FIG. 5 is a view illustrating a coupled state of a needle unit and a transmitter of the analyte monitoring device according to another embodiment of the present invention, and FIG. 6 is a view illustrating a separated state of the needle unit and the transmitter of the analyte monitoring device according to another embodiment of the present invention.

Referring to FIGS. 4 to 6, an analyte monitoring device according to another embodiment of the present invention may include a housing 10, 12, a substrate 30 installed inside the housing 10, 12, a needle unit 20 including a needle body 22 movably installed inside the housing 10, 12 and a needle 24 provided at a lower end of the needle body 22 and inserted into a body to invasively introduce a sensor 42, a first conductive portion 26 disposed on a lower surface of the needle body 22, and a second conductive portion 44 disposed on at least one of an inner side or an outer side of the housing 10, 12 and formed to become electrically conductive in a contact state with the needle unit 20, and when the needle unit 20 is separated from the second conductive portion 44 after triggered, a wake-up trigger for power supply may be transmitted to the substrate 30.

The first conductive portion 26 is provided on a lower surface of the needle body 22 of the needle unit 20. In addition, the second conductive portion 44 which corresponds to the first conductive portion 26, is provided on an inner surface of the lower housing 10. The second conductive portion 44 may be disposed below the needle unit 20 in a vertical downward advancing direction thereof. That is, the second conductive portion 44 may be disposed at a position where the sensing unit 40 is disposed in the above-described embodiment and may have a shape that surrounds the holder 32.

The first conductive portion 26 disposed in this way comes into contact with the second conductive portion 44 and becomes electrically conductive when the needle unit 20 advances. In the case of the analyte monitoring device, before use, the first conductive portion 26 maintains a contact state with the second conductive portion 44, and when used, the needle unit 20 is triggered by the applicator and ultimately separated from the second conductive portion 44.

Meanwhile, the first conductive portion 26 and the second conductive portion 44 may be made of a conductive material. In one embodiment, the first conductive portion 26 and the second conductive portion 44 may be made of conductive silicone rubber. In one embodiment, the first conductive portion 26 and the second conductive portion 44 may be made of a conductive polymer material, a conductive elastomer, a conductive tape, a conductive metal spring pin, carbon nanotubes, or a metal-polymer composite.

As described above, according to the present embodiment, when supplying power to the analyte monitoring device, by utilizing the contact and separation of the sensing unit 40 and the conductive portion 26 or 44, which are made of a conductive material, without applying a semiconductor sensor or a mechanical switch, power may be automatically suppled from a point in time of product use and device durability may be improved during storage and use.

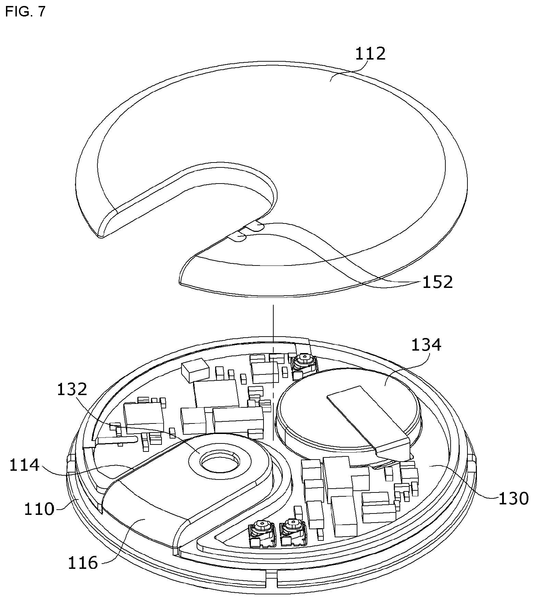

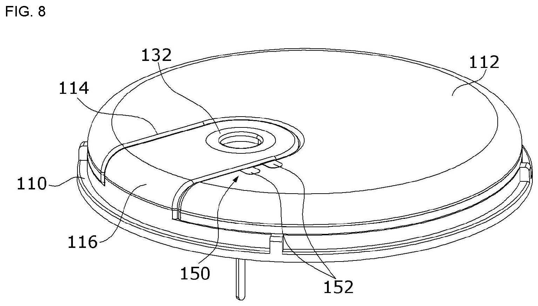

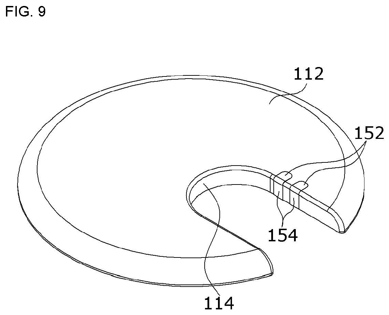

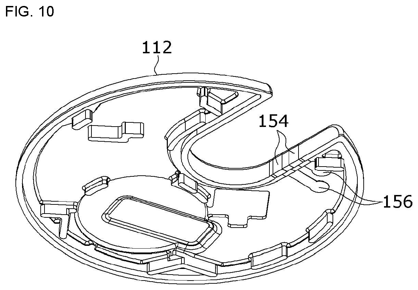

FIG. 7 is an exploded perspective view illustrating an analyte monitoring device according to still another embodiment of the present invention, FIG. 8 is a view illustrating the analyte monitoring device according to still another embodiment of the present invention, FIG. 9 is a view illustrating an upper surface of an upper housing of the analyte monitoring device according to still another embodiment of the present invention, and FIG. 10 is a view illustrating a lower surface of the upper housing of the analyte monitoring device according to still another embodiment of the present invention. In the drawings, the same components as those in the embodiments described above are assigned reference numerals in the 100's, and a detailed description thereof will be omitted for convenience.

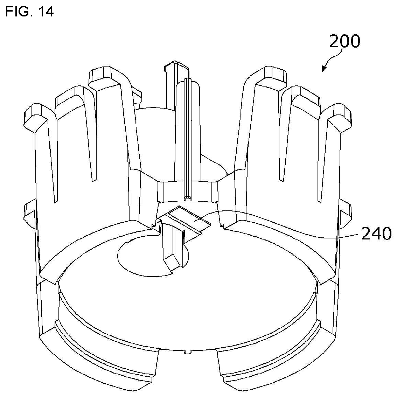

Referring to FIGS. 7 to 10, an analyte monitoring device according to still another embodiment of the present invention may include a housing 110, 112, a needle unit 120 including a needle body 122 movably installed inside the housing 110, 112 and a needle 124 provided at a lower end of the needle body and inserted into a body to invasively introduce a sensor 42 therein, a first terminal portion 150 provided on the housing 110, 112, a transmitter holder 200 (see FIG. 14) to which the needle unit 120 is fixedly installed, which is coupled to an upper portion of the housing 110, 112, and which includes a second terminal portion 240 (see FIG. 14) that comes into contact with the first terminal portion 150 when coupled to the upper portion of the housing 110, 112, and a substrate 130 installed inside the housing 110, 112, electrically connected to the first terminal portion 150, and configured to transmit a wake-up trigger for power supply when separated from the first terminal portion 150 after the needle unit 120 is triggered.

In the present embodiment, a cutout 114 in which a needle coupling portion 116 is disposed is formed in an upper housing 112. The cutout 114 is formed by cutting a portion of the upper housing 112 from the center toward the side and may be cut to have a predetermined width, but is not limited thereto.

The needle coupling portion 116 to which a needle unit 120 is coupled is disposed in the cutout 114. The needle coupling portion 116 is coupled to the side of the lower housing 110 and is positioned in the cutout 114 when the upper housing 112 is coupled to the lower housing 110.

The needle coupling portion 116 is provided with a holder 132 for fixing the needle unit 120 when the needle unit 120 is coupled. As described above, the holder 132 may be provided with a rubber packing to surround and stably fix the needle unit 120.

Meanwhile, the first terminal portion 150 may be disposed across an upper surface, an inner side surface, and a lower surface of the upper housing 112. To be more specific, the first terminal portion 150 may include a 1-1 terminal 152 disposed on the upper surface of the upper housing 112, a 1-2 terminal 154 extending from the 1-1 terminal 152 and disposed on a side surface of the cutout 114 or the inner side surface of the upper housing 112, and a 1-3 terminal 156 extending from the 1-2 terminal 154 and disposed on the lower surface of the upper housing 112.

The first terminal portion 150 is configured as described above so that the first terminal portion 150 is exposed to the upper side of the upper housing 112 and comes into contact with a second terminal portion 240 (see FIG. 14) of a transmitter holder 200 (see FIG. 14). At the same time, the first terminal portion 150, which has come into contact with the second terminal portion 240 (see FIG. 14), is exposed to the lower side of the upper housing 112 and comes into electrical contact with the substrate 130. The first terminal portion 150 may be provided such that a pair of terminals extend in parallel as illustrated in the drawings, but is not limited thereto, and three or more terminals may extend in parallel.

Here, the first terminal portion 150 may be patterned by a laser direct structuring (LDS) method. The LDS method is a method in which a pattern is selectively formed on a thermoplastic resin using a laser and then a plating process is performed to ensure the reliability of electrical characteristics. Meanwhile, the pattern of the first terminal portion 150 is illustrated as a pair being disposed in parallel as described above, but is not limited thereto, and may be formed so that a portion is bent depending on the position of the substrate 130.

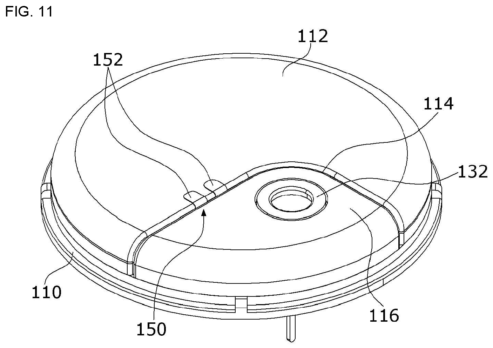

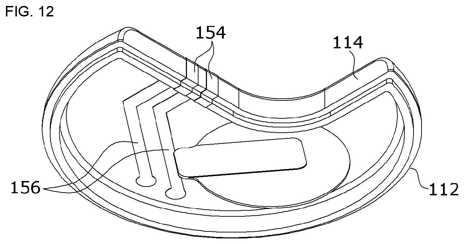

FIG. 11 is a view illustrating an analyte monitoring device according to yet another embodiment of the present invention, and FIG. 12 is a view illustrating a lower surface of an upper housing of the analyte monitoring device according to yet another embodiment of the present invention.

Referring to FIGS. 11 and 12, in the present embodiment, there is a slight difference from the above-described embodiments in the shape of the cutout 114 and the needle coupling portion 116. However, there is no technical difference due to this difference in shape. In the drawings, the needle coupling portion116 may be formed in a fan shape rather than having a constant width but is not limited thereto.

In addition, in the present embodiment, a 1-3 terminal 156 constituting a first terminal portion 150 may be formed so that at least a portion thereof is bent rather than extending in one direction from a lower surface of an upper housing 112. Of course, the shape may vary depending on the position at which the 1-3 terminal 156 is connected to the substrate 130.

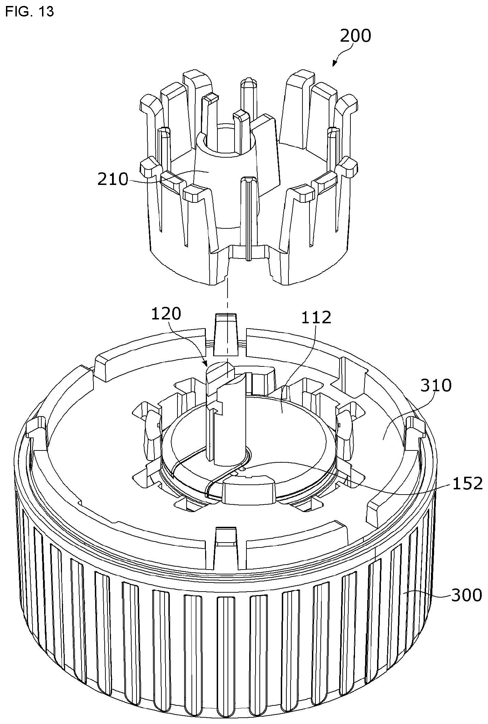

FIG. 13 is an exploded perspective view illustrating an analyte monitoring device according to yet another embodiment of the present invention, and FIG. 14 is a view illustrating a lower surface of a transmitter holder of the analyte monitoring device according to yet another embodiment of the present invention.

Referring to FIGS. 13 and 14, a transmitter holder 200 is formed to be coupled to the housing 110, 112 to linearly move the housing 110, 112 from distal to proximal. Meanwhile, the transmitter holder 200 moves only in a direction from distal to proximal. The transmitter holder 200 includes a holder through-portion 210 through which the needle unit 120 passes.

In addition, a lower surface of the transmitter holder 200 may be provided with a second terminal portion 240 that comes into contact with the first terminal portion 150 when coupled to the upper portion of the housing 110, 112. The second terminal portion 240 may be disposed at a position corresponding to the position of the first terminal portion 150. When the transmitter holder 200 is coupled to the housing 110, 112 in this way, the first terminal portion 150 and the second terminal portion 240 may come into contact with each other to be electrically connected.

In the case of the analyte monitoring device, before use, since the transmitter holder 200 is coupled to the housing 110, 112, the first terminal portion 150 maintains the contact with the second terminal portion 240, and during use, the transmitter holder 200 is triggered by the applicator and ultimately separated from the first terminal portion 150. When separated from the first terminal portion 150, a wake-up trigger for power supply is transmitted to the substrate 130.

A cap 300 is formed to be mounted or detached from a handle (not illustrated) and may protect the interior of the applicator from external contaminants or impact. The cap 300 may be formed in a hollow cylindrical shape, and a cap holder 310 is provided therein. The cap holder 310 serves to align the coupled transmitter holder 200 so that the transmitter holder 200 may be fixed in a correct position.

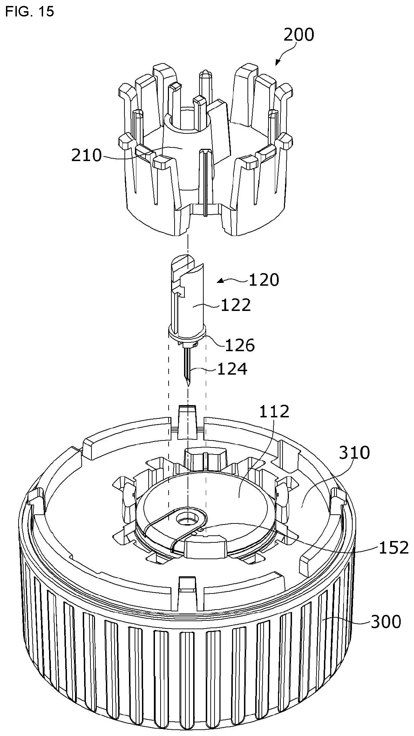

FIG. 15 is an exploded perspective view illustrating an analyte monitoring device according to yet another embodiment of the present invention.

Referring to FIG. 15, in the present embodiment, a second terminal portion 126 that comes into contact with the first terminal portion 150 is provided in the needle unit 120. That is, the second terminal portion 126 may be provided on the lower surface of the needle body 122 constituting the needle unit 120.

When the second terminal portion 126 is provided on the lower surface of the second terminal portion 126 in this way, the second terminal portion 126 comes into contact with the first terminal portion 150 during a coupling process of the needle unit 120. That is, in the present embodiment, unlike the embodiments described above, the terminal contact is not made at the side of the transmitter holder 200 but is made directly at the side of the needle unit 120.

To this end, the second terminal portion 126 may be provided in a circular shape on the lower surface of the needle body 122, but is not limited thereto. In addition, the second terminal portion 126 may have a diameter larger than a diameter of the lower surface of the needle body 122 for contact with the first terminal portion 150. That is, the second terminal portion 126 may protrude radially from the lower surface of the needle body 122 and smoothly come into contact with the first terminal portion 150.

Although specific embodiments of the present invention have been described above, it is understood that one ordinary skilled in the art can make various changes and modifications to the present invention without departing from the spirit and scope of the present invention as set forth in the following claims.

### [Description of the symbol]

10: lower housing 12: upper housing
20: needle unit 22: needle body
24: needle 26: first conductive portion
30: substrate 32: holder
34: battery 40: sensing unit
42: sensor 44: second conductive portion
110: lower housing 112: upper housing
114: cutout 116: needle coupling portion
120: needle unit 122: needle body
124: needle 126: second terminal portion
130: substrate 132: holder
134: battery 150: first terminal portion
152: 1-1 terminal 154: 1-2 terminal
156: 1-3 terminal 200: transmitter holder
210: holder through-portion 240: second terminal portion
300: cap 310: cap holder

## Claims

1. An analyte monitoring device comprising:
a housing;
a substrate installed inside the housing;
a needle unit including a needle body movably installed inside the housing and a needle provided at a lower end of the needle body and inserted into a body to invasively introduce a sensor therein; and
a sensing unit disposed on at least one of an inner side or an outer side of the housing and formed to become electrically conductive in a contact state with the needle unit,
wherein, when the needle unit is separated from the sensing unit after triggered, a wake-up trigger for power supply is transmitted to the substrate.

2. The analyte monitoring device of claim 1, wherein the sensing unit is made of a quantum tunneling composite (QTC).

3. The analyte monitoring device of claim 1, wherein the sensing unit is made in a cylindrical shape or flat shape with a predetermined volume.

4. The analyte monitoring device of claim 1, wherein a holder to which the needle unit is fixed is provided inside the housing, and the sensing unit is provided to surround the holder.

5. An analyte monitoring device comprising:
a housing;
a substrate installed inside the housing;
a needle unit including a needle body movably installed inside the housing and a needle provided at a lower end of the needle body and inserted into a body to invasively introduce a sensor therein;
a first conductive portion disposed on a lower surface of the needle body; and
a second conductive portion disposed on at least one of an inner side or an outer side of the housing and formed to become electrically conductive in a contact state with the needle unit,
wherein, when the needle unit is separated from the second conductive portion after triggered, a wake-up trigger for power supply is transmitted to the substrate.

6. The analyte monitoring device of claim 5, wherein the first conductive portion and the second conductive portion are made of conductive silicone rubber.

7. An analyte monitoring device comprising:
a housing;
a needle unit including a needle body movably installed inside the housing and a needle provided at a lower end of the needle body and inserted into a body to invasively introduce a sensor therein;
a first terminal portion provided on the housing;
a transmitter holder to which the needle unit is fixedly installed, which is coupled to an upper portion of the housing, and which includes a second terminal portion that comes into contact with the first terminal portion when coupled to the upper portion of the housing; and
a substrate installed inside the housing, electrically connected to the first terminal portion, and configured to transmit a wake-up trigger for power supply when the first terminal portion and the second terminal portion are separated after the needle unit is triggered.

8. The analyte monitoring device of claim 7, wherein a cutout in which a needle coupling portion to which the needle unit is coupled is disposed is formed in the housing.

9. The analyte monitoring device of claim 8, wherein a holder for fixing the needle unit is provided in the needle coupling portion.

10. The analyte monitoring device of claim 8, wherein the housing includes a lower housing and an upper housing coupled to an upper portion of the lower housing, and
the first terminal portion is disposed across an upper surface, an inner side surface, and a lower surface of the upper housing.

11. The analyte monitoring device of claim 10, wherein the first terminal portion includes:
a 1-1 terminal disposed on the upper surface of the upper housing;
a 1-2 terminal extending from the 1-1 terminal and disposed on a side surface of the cutout; and
a 1-3 terminal extending from the 1-2 terminal and disposed on the lower surface of the upper housing.

12. The analyte monitoring device of claim 11, wherein the 1-3 terminal is electrically connected to the second terminal portion.

13. The analyte monitoring device of claim 7, wherein the first terminal portion is patterned by a laser direct structuring (LDS) method.

14. An analyte monitoring device comprising:
a housing;
a needle unit including a needle body movably installed inside the housing and a needle provided at a lower end of the needle body and inserted into a body to invasively introduce a sensor therein;
a first terminal portion provided on the housing;
a second terminal portion provided on a lower surface of the needle unit and electrically connected to the first terminal portion; and
a substrate installed inside the housing, electrically connected to the first terminal portion, and configured to transmit a wake-up trigger for power supply when the first terminal portion and the second terminal portion are separated after the needle unit is triggered.

15. The analyte monitoring device of claim 14, wherein a cutout in which a needle coupling portion to which the needle unit is coupled is disposed is formed in the housing.

16. The analyte monitoring device of claim 15, wherein the housing includes a lower housing and an upper housing coupled to an upper portion of the lower housing, and
the first terminal portion is disposed across an upper surface, an inner side surface, and a lower surface of the upper housing.

17. The analyte monitoring device of claim 16, wherein the first terminal portion includes:
a 1-1 terminal disposed on the upper surface of the upper housing;
a 1-2 terminal extending from the 1-1 terminal and disposed on a side surface of the cutout; and
a 1-3 terminal extending from the 1-2 terminal and disposed on the lower surface of the upper housing.

18. The analyte monitoring device of claim 17, wherein the 1-3 terminal is electrically connected to the second terminal portion.

19. The analyte monitoring device of claim 14, wherein the first terminal portion is patterned by a laser direct structuring (LDS) method.
